# EUROPEAN PATENT APPLICATION

(11) **EP 2 589 401 A1**
(43) Date of publication of application: **08.05.2013**
(21) Application number: 11187847.6
(22) Date of filing: 04.11.2011
(51) Int. Cl.: A61M 5/42, A61M 5/46, A61M 5/32

(54) **Device for controlling the depth and reducing the pain of injections**

(71) Applicant: Fiderm S.r.l. (Ricerca e Technogie in Scienze Dermatologiche), 20129 Milano (IT)
(72) Inventor: Di Pietro, Antonino, 20064 Gorgonzola (IT); Apolet, Josek Berek, 20146 Milano (IT)
(74) Representative: Palladino, Saverio Massimo

(57) **Abstract**

The present invention relates to a device (10) to be used on a needle holder or on a syringe for injection, which permits a reduction of the pain due to the injection as well as the control of its depth.

## Description

### Field of the invention

The present invention relates to a device to be used with syringes for injection, which permits a reduction of the pain due to the injection as well as the control of its depth, even in the case of serial multi-injection.

### Prior art

It is known how to perform injections of substances and compositions of various types into the human body (for medicinal and/or cosmetic purposes) and into animals (for veterinary purposes); for this purpose, needles of different lengths and diameters, mounted on syringes, are used. Substances that are injected for medicinal purposes are generally all active substances or compositions, commonly in the form of liquids or solutions; substances that are injected for cosmetic purposes may be fluids, solutions (for example, aqueous solutions of hyaluronic acid for the remodelling of wrinkles) or even gaseous substances, such as ozone or carbon dioxide used in the treatment of cellulite.

The depth of injection varies depending on the type of substance injected and the purpose of the injection.

Currently, for injections for medicinal purposes, it is preferred to perform so-called intramuscular injections, i.e. in which the tip of the needle reaches a point within a muscle bundle; for this purpose, needles are used that are generally greater than 2 cm in length, typically between 2.5 and 3.8 cm, although longer needles are also used for patients in whom the layer of subcutaneous fat covering the muscle is particularly thick.

Subcutaneous injections, i.e. injections with a depth of around 1 cm, are used in particular in the case of vaccinations, insulin therapy, antithrombotic therapy, etc.

Injections for cosmetic purposes, on the other hand, are normally performed intradermally, i.e. into the thickness of the skin, for example around 4-6 mm below the skin surface, in the area of the mesoderm.

In the case of subcutaneous injections and, in particular, intradermal injections, controlling the depth of injection is particularly critical, given the reduced thickness of the usable area. This problem can be solved with devices for controlling the depth of the injection, such as the one described in patent EP 1045710 B1 in the name of one of the present inventors.

A problem common to all injections is the pain caused to the patient. The problem can in some cases be alleviated, at least in part, through the use of very thin needles; however, needles that are too thin are not suitable for the injection of particularly viscous fluids, such as dense solutions, oily substances or compositions in the form of a suspension.

The pain due to injections is also a problem in the case of intradermal injections with thin needles, because of the need to perform several injections in a short time span in a limited area of the body (a technique called "picotage").

Injections also have other undesirable consequences.

Firstly, all forms of injection used today at least reach the mesoderm area, where there are nerve endings and which is irrigated by small blood vessels. The presence of the nerve endings, which can be touched by the needle, is the cause of the pain during injections. The blood vessels may also be cut by the needle, causing local ecchymoses or haematomas and inflammation due to the repair phenomena implemented by the body in response to any trauma.

In addition, the rate of absorption of the injected substance is not always optimal. Even in the case of intramuscular injections, which are characterised by a relatively fast absorption thanks to high blood flow in the muscles, it is possible that deposits are formed, in particular when oily fluids are injected; the complete absorption of the injected fluid can sometimes take up to several days, causing pain to the touch in the affected area.

One purpose of the present invention is to provide a device that permits a reduction of the pain caused by injections as well as the control of their depth at such a level as to reduce some of the undesirable side-effects and to maximise the rate of absorption of the injected fluids.

### Summary of the invention

These and other purposes are achieved with the present invention, which in a first aspect relates to a device to reduce the pain caused by injections, while at the same time controlling the depth of the injections.

The device according to the invention, which is applied to a syringe or to the needle holder of a syringe and is intended to come into contact with the skin during an injection, consists of a hollow body having an essentially axial symmetry and that, in a sectional view along the axis of symmetry, shows in order: a first part shaped internally so as to be able to be firmly attached to a needle holder, a syringe, or an adapter attached to a needle holder or a syringe; a second central part, elastically deformable between two fixed positions of maximum and minimum extension, connected to said first part; and a third part, connected to said central part, consisting of at least three lobes facing inwards, separated from each other by grooves and having an opening on the part opposite to said central part; said device being such that, at rest, said central part presents a first axial length **H** and said opening presents a first diameter value **D,** while when the device is compressed, said central part presents a second axial length **h < H** and said opening presents a second diameter value **d < D.**

With regard to pain control, the device of the invention takes advantage of a principle described in the so-called "Gate Control Theory" or GCT, formulated by R. Melzack and P. Wall in 1962. According to this theory, tactile stimuli "travel" along the nerve fibres faster than the pain signal; since both signals are received initially by spinal cord neurons (and, from there, are sent to the brain), it is believed that a spinal cord neuron that is already "engaged" by a tactile stimulus is unreceptive (or receptive to a lesser degree) to the pain stimulus; as a result, the stimulation of the nerve fibres that transmit the tactile stimuli inhibits the cells intended for the reception of the pain stimuli, thus reducing or inhibiting the transmission of pain. With the use of the device of the invention, at the time of the injection the operator presses the needle towards skin, thus deforming, as detailed below, both the central elastic part and the lobed part of the device; the deformation of preset entity of the elastic part guarantees the precision of the depth of the injection, while the deformation of the lobed part, which occurs before the needle penetrates the skin, causes a lateral compression around the injection site that causes a reduction of pain.

### Brief description of the figures

- Figure 1 shows the device according to the invention in its most general form;
   in the figure, the device is shown in a schematic view in cross-section, when it is not in use (on the left side of the figure) and when it is in use (on the right side of the figure);
- Figure 2 shows the device of Figure 1 in a perspective view;
- Figures 3.a, 3.b and 3.c show some possible embodiments of the device according to the invention, which differ from each other in terms of the construction of the central elastic part;
- Figures 4.a, 4.b and 4.c show various possible embodiments of the first part of the device according to the invention, suited to being mounted in a fixed position on a needle holder or syringe;
- Figure 5 shows a different embodiment of the device according to the invention, wherein the device is made of a single part with the needle holder;
- Figures 6, 7 and 8 show various possible embodiments of the device according to the invention, suited to being mounted in an adjustable position on a needle holder or syringe;
- Finally, Figures 9.a, 9.b and 9.c show a further embodiment of the invention, wherein a device according to the invention is mounted on a needle holder by means of an adapter.

### Detailed description of the invention

In the following description, reference is made to the device according to the invention as formed by the set of three "parts"; however, this definition is given solely for the purpose of clarity of description and, as will become apparent to the person skilled in the art, in practical embodiments these "parts" may simply be the different areas of a device made of a single part.

In the figures, the different parts of the device of the invention are not necessarily shown to scale; in particular, in some cases, the thickness of the parts can be increased for reasons of clarity of design; the same numbers in different figures indicate identical or corresponding elements.

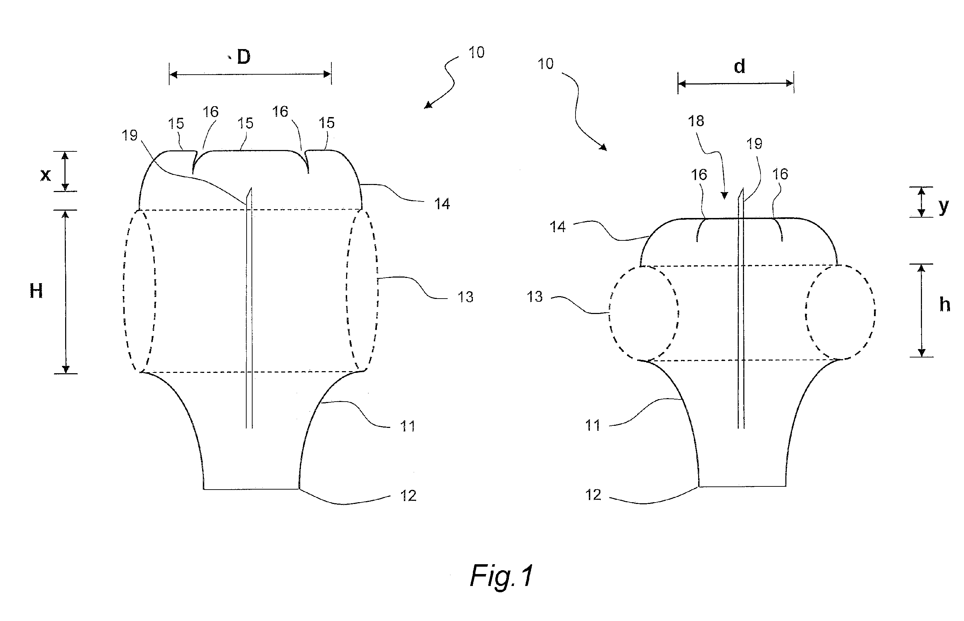

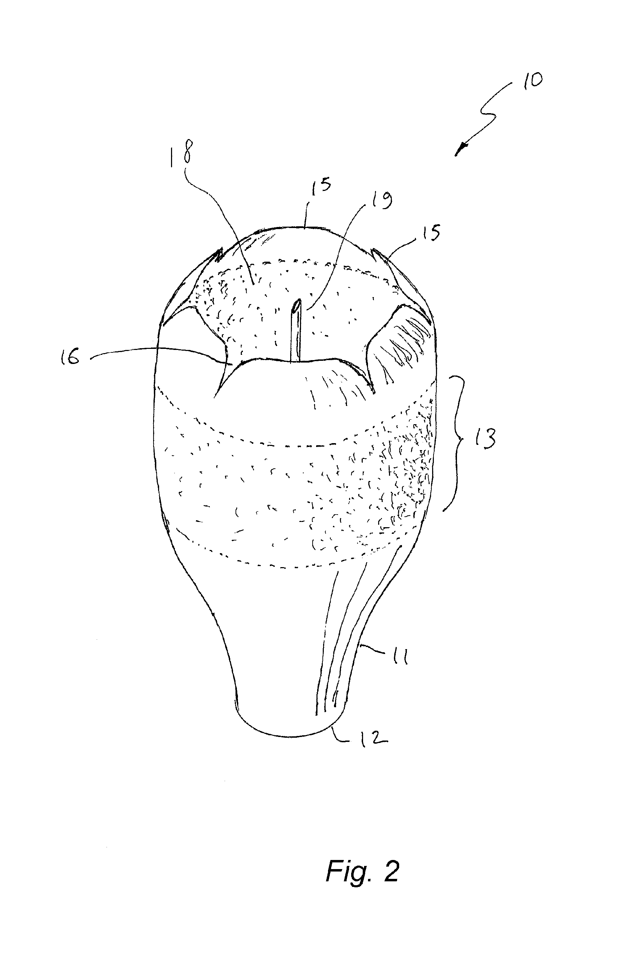

In its most general embodiment, the device according to the invention is shown in Figures 1 and 2; the left part of Figure 1 illustrates the device of the invention when it is not in use (and therefore not subjected to axial compression), while the right part of the figure illustrates the device during injection (compressed in the axial direction).

The device of the invention, 10, comprises a first part, 11, shaped in its interior and at a first end, 12, so that it can be solidly fixed to a needle holder or syringe; starting from the end 12 and moving along the axis of the device, the part 11 increases in its section until it connects with the second central elastic part, 13, schematically represented in Figure 1 with an ellipsoidal dotted line and in Figure 2 as an area delimited by the two dotted lines; finally, there is the third part, 14, intended to come into contact with the skin during the injection, which is divided into lobes 15 separated from one another by grooves 16. Figure 1 also shows the position of the needle 19 relative to the device under the two conditions (not in use and during injection), to highlight the operation of the device, without however going into details of how the needle is fixed to the syringe or to the needle holder to which the device 10 is connected.

The third part of the device has a constant geometry in all possible embodiments of the device, while the first and second parts can be implemented in different ways. The three parts are described separately below; it will be apparent to the person skilled in the art that a device of the invention can be produced by combining the third part with a first part having any of the geometries or design solutions described below, and with a second part having any of the geometries or design solutions described below.

The third part, 14, of the device according to the invention has a series of lobes, 15; these must number at least three, so as to be able to perform the action of lateral compression described below, while their maximum number is dictated by reasons of convenience, because an excessive number of lobes would lead to edges of much reduced length, which could cause pain; in the practical embodiments, the device of the invention has preferably between six and twelve lobes. The lobes 15 are bent towards the inside of the cavity of the device, preferably at an angle of between 30° and 60°.

The external edge of each lobe 15 is the part intended to come into contact with the skin during the use of the device, and is arranged on a circumference perpendicular to the axis of the device; the arrangement of the edges of the lobes 15 on said circumference creates a front opening, 18, of the device. Between two adjacent lobes there is a groove, 16, that is wider towards the edge of the lobe and having a width that gradually decreases towards the central part 13 of the device; the shape of the lobe is such that the groove and the edge are connected by a curve, thus preventing sharp edges that could themselves cause pain to the patient. With this geometry, the edges of the lobes form a discontinuous track on said circumference, and therefore on the skin of the patient when the device is at rest.

The second part, 13, has the characteristic of being elastic and being able to be deformed between a position of maximum extension and a position of minimum extension following the compression exerted during the injection. In the device at rest (left side of Figure 1), the part 13 has a first height, **H** (maximum extension), the grooves 16 are open, thus keeping the lobes 15 separated from each other, the edge of the lobes 15 is arranged on a circumference which has a first diameter value, **D,** and the tip of the needle 19 does not emerge from the opening 18 and is located at a distance **x** from the level of said circumference. During the injection, the device 10 is compressed between the syringe or needle holder on which it is fixed and the area of skin in which the injection is to be performed; as a result of this compression (right side of Figure 1), the part 13 undergoes a shortening in the axial direction of the device (and in some cases an enlargement of its cross-section) and comes to have a second height **h** (minimum extension, **h < H**); the shortening of the device 10, equal to the distance (**H - h**), causes the tip of the needle 19 to emerge from the opening 18 by a length **y.** Clearly, the relationship between the various axial measures mentioned above is (**x + y**) **=** (**H - h**); thus, once it is known what shortening (**H - h**) the device undergoes during use (shortening that can be preset precisely at the production site of the device), and once it is known the initial positioning of the needle relative to the device 10, which determines the value of **x,** it is possible to precisely preset the value of **y,** and therefore the depth of injection under the skin surface of the patient. At the same time as the shortening equal to the value (**H - h**), the lobes 15 move closer to each other, until the closure of the grooves 16, and their edge comes to be located on a circumference which has a second diameter value, **d,** which is less than **D;** in this way, the lobes 15 exert a radial compression on the skin area surrounding the injection site and thus cause a reduction of the pain. The limit of this inward movement (the closure of the grooves 16) is preset at the production site of the device 10.

The central part 13 can be designed in several different ways.

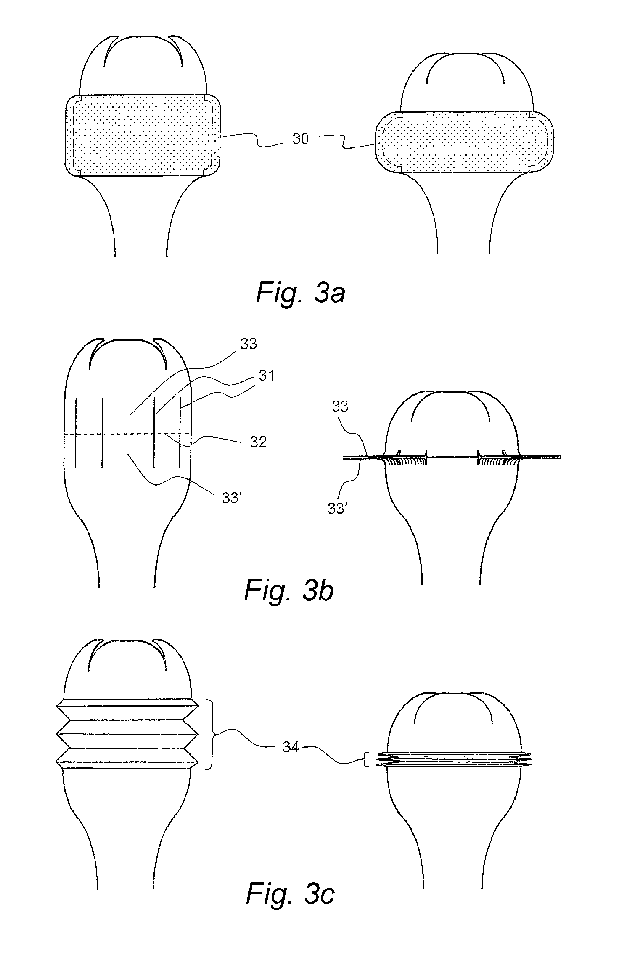

A first possible design is shown in Figure 3.a. In this embodiment, the elastic central part consists of an elastic deformable strip, 30. This strip is shaped so as to present, in a sectional view, a concavity toward the inside of the device and a convexity towards the outside of the device (the dotted lines in the figure represent the internal surface of the strip 30, in a sectional view). With this geometry, as a result of an axial compression, the strip 30 undergoes an outward expansion, as shown in the figure, and a shortening in the axial direction; the left side of the figure shows the device at rest, with the part 13 in the maximum extension arrangement, and the right side shows the device in compression, with the part 13 in the minimum extension arrangement, which causes the emergence of the needle from the opening between the lobes 15.

A second possible design is shown in Figure 3.b. In this embodiment, the central part has a structure of the "Malecot catheter" type, i.e. a cylindrical part with a series of cuts, 31, parallel to each other and to the axis of the cylinder (and preferably equidistant), with predefined fold lines 32 transverse to the cuts (shown as dotted lines on the left side of Figure 3.b). In this way, parallel "bands" are formed from parts 33 and 33', above and below the fold lines. Also in this case, the left side of the figure shows the device at rest, while the right side shows the arrangement assumed by the device after compression in the axial direction, with the areas of part 13, separated by the cuts 31, which are folded towards the outside along the lines 32, thus causing the shortening of extent (**H - h**) of the device 10.

Finally, Figure 3.c illustrates another embodiment of the central part 13, wherein this part has the shape of a bellows, 34. Also in this case, the left side of the figure shows the device at rest, while the right side shows the arrangement assumed by the device after compression in the axial direction.

The bellows of Figure 3.c is the preferred embodiment of the present invention; for this reason, in the rest of the description and in the associated figures, reference will be made in particular to devices with the central part 13 consisting of a bellows 34, but it remains understood that everything described below could be produced with any central part that has the above-described characteristic of being able to be compressed elastically between two extreme positions, corresponding to the heights **H and h,** such as the central parts illustrated in Figures 3.a and 3.b.

The first part of the device can be produced according to various embodiments, depending on whether it is mounted directly on a syringe or on a needle holder of the same, on the geometry of the mounting position, and on the type of mounting, which can be fixed or adjustable; in all of these alternative forms, in general, the external appearance of the first part will not change, and what changes is its internal shape, which must be complementary to the shape of the needle holder or the syringe on which the device 10 is mounted.

In the most common method, the mounting occurs on a needle holder, but there are syringes (in particular of small diameter) in which the use of a needle holder is not foreseen, and in which the needle emerges directly from the bottom wall of the syringe. The mounting methods described below refer to the most common case of mounting on a needle holder, but the same can also be adopted for direct mounting on suitably shaped syringes. In the event that the device of the invention is connected to a needle holder, and this is in turn mounted on a syringe, the needle holder can be mounted on the syringe with fastening methods known in the industry, in particular with "cone" fastening or fastening of the Luer-lok type.

According to a first possible alternative, the mutual position of the device according to the invention and the syringe or needle holder is fixed. According to this embodiment, the device can be mounted by the operator immediately before use, or it can be produced in a single body with the syringe or needle holder.

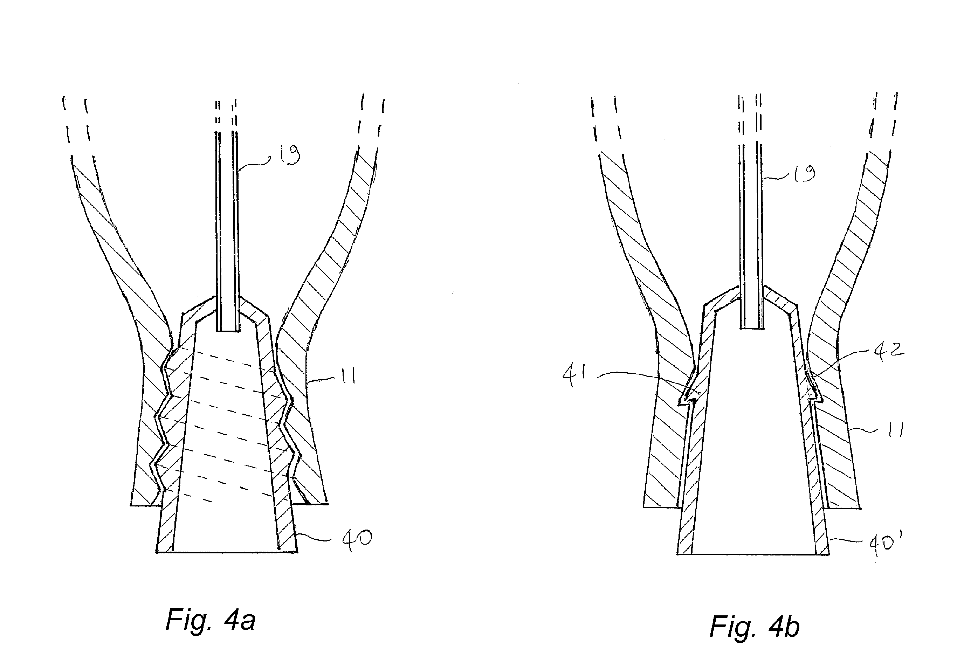

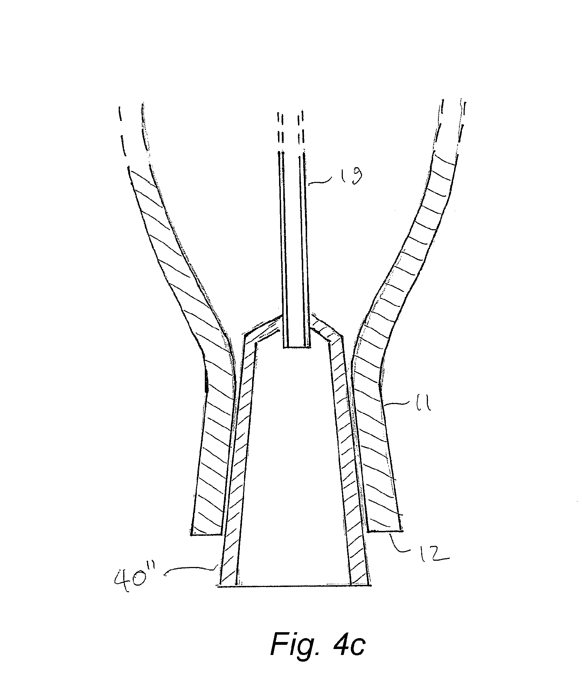

Various alternative ways of mounting a device of the "discrete" type on the needle holder, in a fixed reciprocal arrangement, are illustrated in the drawings of Figure 4; these drawings show only the part 11 of the device 10 and the means of connection of the latter to the needle holder.

A first possible fixed mounting can be made by screwing, by suitably threading the interior of the part 11 and the exterior of the area of the needle holder 40 on which the device 10 must be mounted, as shown in a sectional view in Figure 4.a (the thread is shown by the dotted lines). In the figure, the lateral surface of the needle holder is shown with a truncated conical geometry (the most common), but this could simply be cylindrical. In the case shown in the figure, truncated conical surfaces, the development of the thread will follow the main geometry, and the diameter of the helix of the thread will be narrower in the part from which the needle emerges and wider in the part where the needle holder is connected to the syringe; in this way, the shape of the parts 11 and 40 and the threads themselves determine the end point of the screwing movement of the two parts.

The mounting can be performed by interlocking, providing in this case the presence of suitable elements in the device and the needle holder, such as teeth or protuberances and corresponding recesses or grooves. Preferably, these elements are of the "non-return" type, i.e. such that the reciprocal sliding of the two parts at the time of mounting is easy, but very difficult in the opposite direction once the mounting is completed, and such that, to separate the two parts, the breakage of one of the two is generally required. A possible mounting of this type is shown in Figure 4.b, wherein the external surface of the needle holder 40' has an annular protuberance 41 with a triangular section, which fits into a groove 42 of corresponding shape in the internal surface of the part 11 of the device 10 (the reverse situation is also possible, wherein the protuberance is on the inside of part 11 and the groove is on the external surface of the needle holder). The sliding of the device 10 up to the point where said protuberance is inserted into the groove is made possible by the elasticity of the polymer materials the device 10 (and in most cases also the needle holder) is made of; this elasticity enables the part 11 to expand sufficiently until the protuberance 41 reaches the groove 42, after which the same elasticity determines the insertion of the protuberance into the groove, and the geometry of these two elements is such to prevent the reverse movement.

Another possibility is to fix the device to the needle holder by simple friction (as in the case of mounting a needle holder on a syringe known in the industry as a "Luer slip"). In this case, illustrated in Figure 4.c, part 11 of the device has, on its inside, a truncated conical shape, with the widest part of the cone at the end 12, and corresponding to the shape of the part of the needle holder 40". By exerting pressure during the mounting of the device 10 on the needle holder, possibly by heating one or both of the parts, an adhesion by friction is achieved that is sufficient to ensure that the device 10 remains integral with the needle holder during the injection or series of injections (in the case of picotage). In this case, it is preferable to provide an endpoint system between the two parts, for example, an abutment on one of the two that is to rely on an appropriate reference (a prominent ring or step, a series of bumps,..) on the other; this first ensures a more solid mounting of the two parts, and secondly, it ensures the reciprocal positioning of device 10 with respect to the needle holder, and consequently to what extent the needle comes out from the device during an injection (and thus the reproducibility of the depth of injection).

In figures 4.a, 4.b and 4.c, the parts 11 and 40, 40' or 40" are shown slightly spaced apart, for the sole purpose of highlighting the design details of the same and their details (for example, the protuberance 41 and the groove 42), but it is clear that in reality these parts are in close contact with each other. Moreover, in these figures, the dotted line in the upper part of the first part 11 indicates that this continues towards the area wherein it connects or joins to the central part 13 (not shown).

The device 10 can also be fixed to the needle holder with adhesives (case not shown in a figure).

Finally, the device according to the invention can be connected to the needle holder with a Luer-lok type connection, which is well known in the field of injection syringes for connecting the needle holder to the main body of the syringe. To use this type of connection, the needle holder is integral with a cylindrical part that surrounds it, forming an essentially cylindrical "throat"; the cylindrical part around the needle holder has a thread on its internal surface, and the external shape of the part 11 of the device 10 will have the corresponding counterthread and such dimensions to fit between the needle holder and said internally threaded cylindrical part. The stopping point of the screwing can be determined by the contact between the end 12 of the device 10 and the bottom of said "throat", or a suitably designed system, such as a tooth or a raised ring present on one of the two parts to be fixed, and a recess with a shape corresponding on the other part.

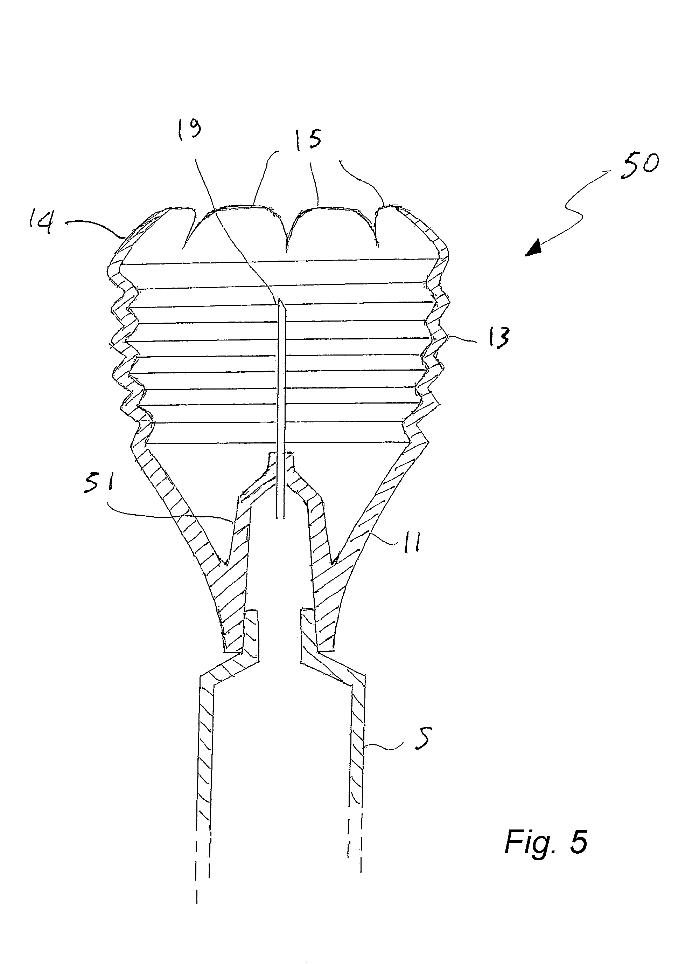

The fixed mutual positioning between the device according to the invention and the needle holder (or syringe) can also be obtained in the manner illustrated in Figure 5. This type of the device, 50, is such that at least its first part 11 is designed as a single body with the needle holder, 51; the assembly 50 is then mounted on the syringe, S, in a manner known in the field. In the case illustrated in Figure 5, the entire device 50 is made of a single part, but as mentioned above (case of Figure 3.a), the part 13 could be a separate part connected to the parts 11 and 14; besides, it is illustrated the case of a device in a single part with the needle holder, but in an analogy to what was said before, for the direct mounting of the device of type 10 on a syringe, also device 50 could be made as a single part with the body of the syringe.

In the embodiments described above, with a fixed mutual positioning between the device according to the invention and the needle holder (or syringe), the depth of penetration of the needle during the injection is also fixed; it is possible to provide for the construction of devices of type 10 or 50 with different dimensions, and in particular with different lengths along the axis thereof, to make it possible for doctors to give injections at different depths. Devices for different depths of injection can be distinguished, for example, by producing them in different colours, or by using other indicators, such as coloured lines, raised lines or the like, each corresponding to a different height of the device.

In another alternative, the mutual positioning of the device of the invention and the needle holder (or syringe) is adjustable; in this second form too, it is possible to provide for the mounting of the two parts being performed by the operator immediately before the injection (or series of injections), or the device and the needle holder can be produced and sold in an already assembled form. The construction required to obtain an adjustable mutual positioning of the device and the needle holder (or syringe) is more complex and expensive than in the case of fixed mutual positioning, but offers the advantage of giving the operator the possibility to decide the distance between the level of the opening 18 and the tip of the needle 19 (i.e. having a variable distance **x**), and consequently the depth of penetration of the latter, depending on the type of therapy intended.

The mutual adjustment of the positioning of the device in relation to the needle holder (or syringe) may be discrete or continuous. While in the case of "fixed length" mountings described above, for example with reference to Figures. 4.a, 4.b and 4.c, the needle holder could have an external surface that runs primarily cylindrically or in a truncated conical fashion (short of threads, teeth, grooves, etc.), in cases of adjustable mutual positioning, the external surface of the needle holder (or syringe) and the internal surface of the device of the invention must necessarily have a primarily cylindrical course, so that the two parts remain in close contact with each other while sliding reciprocally in the axial direction.

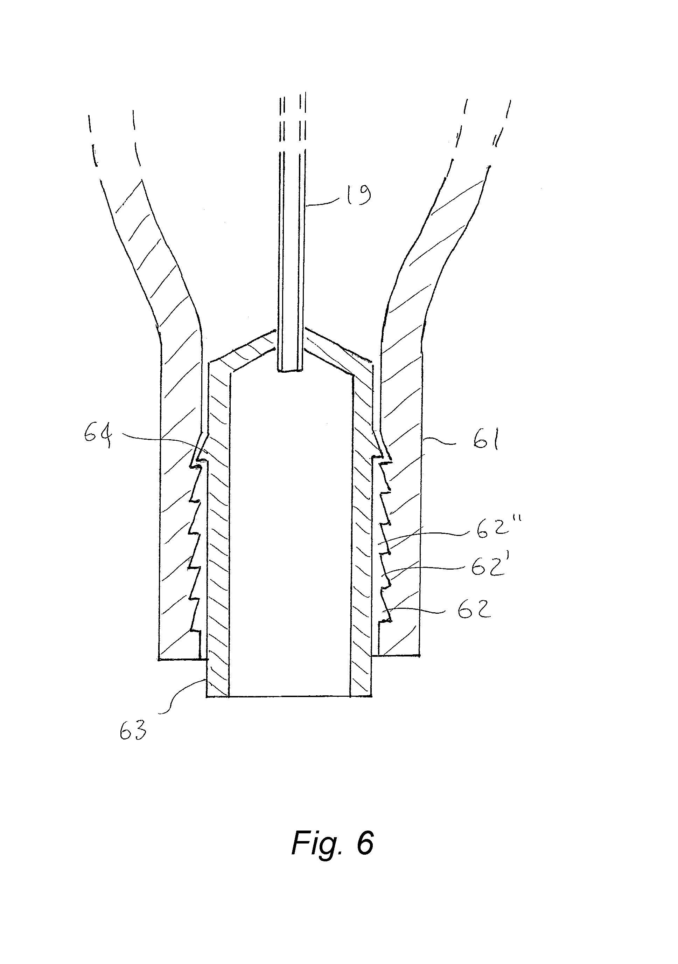

A discrete adjustable mounting (i.e. at various preset mutual positions device/needle holder) can be obtained for example with the arrangement shown in Figure 6. In this case, the first part of the device, 61, presents a series of annular grooves 62, 62', 62",..., similar to the groove 42 of Figure 4.b, and the needle holder, 63, has a protuberance 64 similar to protuberance 41; the part 61 (and hence the device of the invention) can be moved respective to the needle holder so that the protuberance 64 fits into any of the grooves 62, 62', 62",..., determining the mutual positioning of the two parts.

Continuously adjustable mountings (between two extreme positions) can be obtained in several ways.

One possibility is to create the mounting with external threads on the tip of the needle holder (or syringe) and counterthreads on the internal surface of the first part of the device; if the two parts are in close contact, it is possible to move the device along the thread by exerting a tangential force, and thus moving it axially to the desired length, but the friction between the two parts will be such to prevent further movements when the elastic part of the device is stressed axially during the injections.

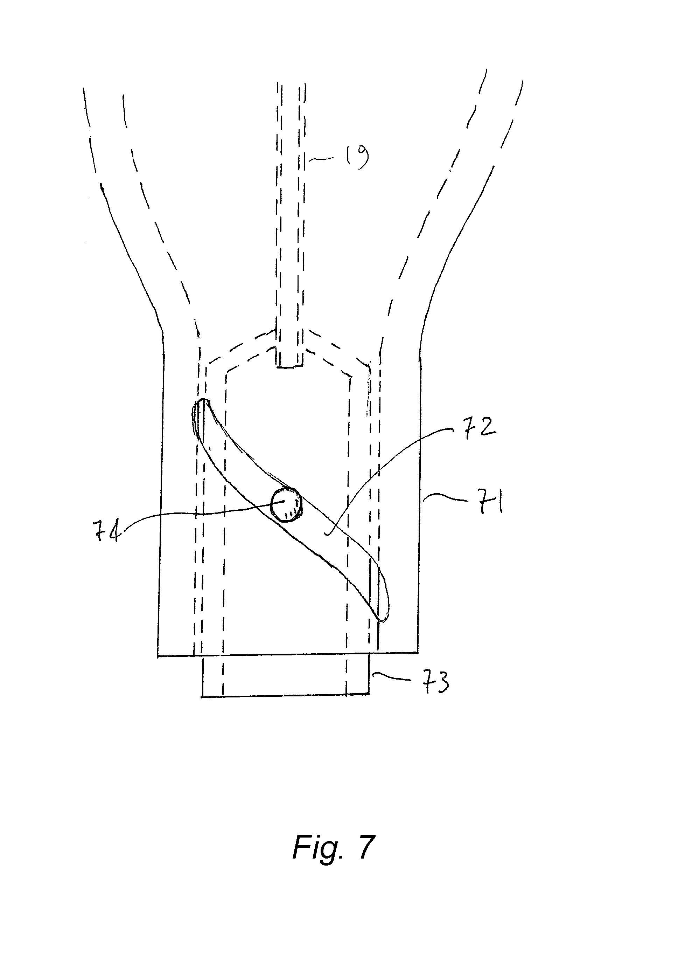

Alternatively, it is possible to provide a mounting (schematically illustrated in Figure 7) wherein the first part 71 of the device of the invention has an oblique groove 72 (shown in the figure as a pass-through, i.e. also open to the outside of the device, but which could also be only an indentation on the internal surface of the part 71), and the needle holder 73 has on its external surface a protuberance 74 (known as the "mushroom" in the industry) that slides into the groove 72 (it is also possible to produce the protuberance on the internal surface of the part 71 and a groove, not a pass-through one, on the external surface of the needle holder); the figure shows only one groove 72 and only one protuberance 74, but there are preferably two pairs of these elements, arranged symmetrically around the axis of the system. Also in this case, protuberances and grooves are produced with dimensions so as to exert friction against each other, so that the part 71 can be moved respective to the needle holder 73 by exerting a manual rotation effort, but it does not move during the injections.

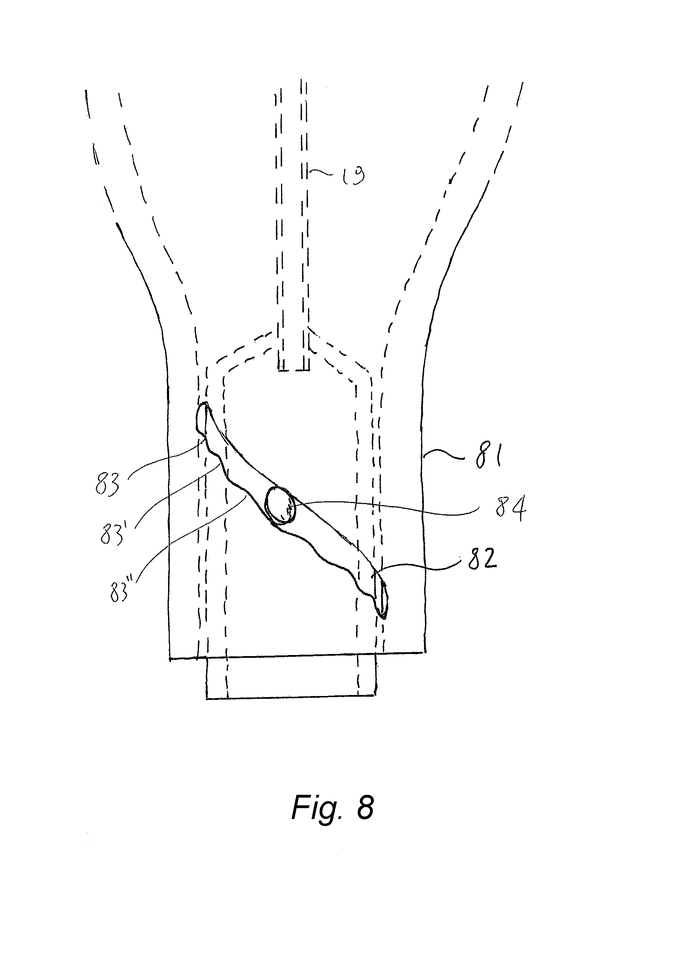

The embodiment illustrated in Figure 7, which allows a continuous adjustment of the depth of injection between two preset values (and defined by start and end points of the groove 72), can also be adapted to give rise to a discrete adjustment. This case is illustrated in Figure 8. In this form, the first part 81 has a groove, 82, having a non-constant width but rather a number of minor equidistant narrowed sections, 83, 83', 83",..., that subdivides it into housings with a section equal to the "mushroom" 84; the passage of the "mushroom" from one of these openings to the next one requires the temporary deformation of the same, which is possible thanks to the elasticity of the polymer material from which it is made, and is possible by exerting at the same time on the device a rotational force and a force in the axial direction; the simple axial stress that occurs during the injection is not sufficient instead to move the "mushroom" from one housing to the next.

In Figures 7 and 8, the first part 71 or 81 is not shown in cross-section, in order to highlight the groove 72 or 82; therefore, all of the hidden details of the first part (the needle holder, the section of the first part, the needle 19) are shown by dotted lines to indicate that they are hidden from the viewable face of said first part.

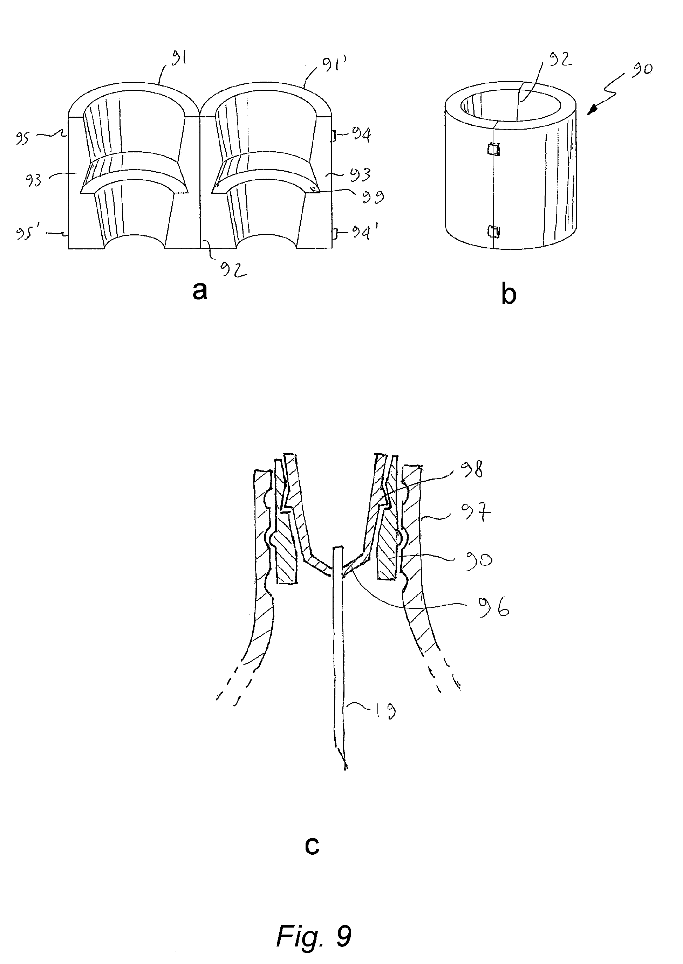

Finally, in a preferred embodiment, the device according to the invention can be fixed to the needle holder (or syringe) by means of an adapter interposed between the two. This embodiment is schematically illustrated in Figure 9; Figure 9.a shows the adapter in the open configuration, Figure 9.b shows the closed adapter, and Figure 9.c shows the assembly consisting of a device of the invention mounted on a needle holder by means of the adapter. The adapter, 90, is composed of two semicylindrical parts 91 and 91' joined together, for example, by means of a line, 92, wherein the material of the adapter has a reduced thickness; the adapter can thus be folded around this line, resulting in a configuration of the "book" type. Each of the two main parts of the adapter 90 is internally shaped to complement the needle holder (or syringe) on which it is to be fixed; even in this case, it is possible to provide for the presence of internal elements (teeth, protuberances, grooves, ...) to be matched to corresponding elements on the external surface of the needle holder (or syringe); Figure 9.a shows a groove 99 with a triangular section, suited to engaging with the protuberance 98 on the needle holder shown in section in Figure 9.c. By folding the two parts around the line of reduced thickness, it is possible to match their two outer sides, 93 and 93', forming the adapter 90. Said outer sides have elements, 94, 94' and 95, 95', which can be fixed to each other, thus closing the structure.

At the time of use, the adapter 90 is closed tightly around the needle holder (or syringe); the construction of the adapter 90 is such that, when closed around the needle holder 96 (or syringe), it cannot move axially along the latter (due to friction or to the presence of retention elements as described in previous embodiments). The adapter 90 also has protuberances on its external surface, which could also simply be the two protrusions formed by the closure of the elements, 94, 94' and 95, 95', which are inserted into one of several possible grooves on the internal surface of the first part, 97, of the device of the invention. The elements 94, 94' and 95, 95' are such that, once connected to each other (and thus when the adapter 90 is closed), they can no longer be reopened without causing them to break. In this way, it is possible to fix the device of the invention at one of the several possible heights provided, but only in an irreversible way, i.e. without the possibility of reopening the assembly to re-use it later on, thus ensuring that the device can be used for a single injection or at most a series of injections (in the case of "picotage"), and thus ensuring the hygiene of the system.

The device of the invention can be produced in general with any plastic material that has sufficient mechanical strength (taking into account the thickness of the specific device to be produced); preferred materials for the construction of the device are polyethylene, polypropylene, polyamides or copolymers such as ethylene-vinyl acetate.

The thickness of the material forming the device of the invention is not binding, and it is sufficient for said thickness to guarantee the rigidity required so that the device does not deform during the injection, except for the compression of the part 13 and the inward bending of the lobes 15, both by the extent preset at the production site by the geometry of the device. Typically, the wall thickness of the device 10 is comprised between about 0.2 and 1 mm, and can vary within the same device; for example, the thickness is generally greater at the end 12, in order to ensure the rigidity of the first part 11 (and thus the strength of the fitting on the syringe and the coaxiality with the same), while it may be reduced in the area of the lobes 15.

The overall dimensions, and in particular the axial length of the device of the invention, are variable depending on the length range (**H - h**), and therefore on the depth of injection, that is desired to be obtained. The exact dimensioning of the device depends on the particular constructing geometry followed, for example on the greater or lesser length of the three parts that make it up and on the choice of the second part; in fact, a second part designed in the manner shown in Figure 3.b ("Malecot" structure) allows the maximum length reduction, a second part designed according to the method of Figure 3.a generally undergoes a lesser shortening (**H-h**), while a second part with a bellows (structure in Figure 3.c) has a shortening that is intermediate between the two previous cases. For intramuscular injections, as mentioned in the introduction, it is necessary to reach injection depths of at least 2 cm, and preferably between about 2.5 and 4 cm; to obtain these injection depths, also taking into account that in the device of the invention at rest the tip of the needle is at a distance **x** from the opening 18, the device will generally have an overall length of at least 7-8 cm, while in the case of subcutaneous injections (injection depth of around 1 cm), the device can have an overall length of less than 5 cm.

In a preferred embodiment, the dimensions of the device according to the invention are such that, when this is fixed to a syringe, the injection of the fluid is made at a minimum depth, in particular just below the epidermis. The epidermis is the outermost layer of skin, and is not vascularised nor it contains nerve endings; capillaries and nerve endings are in fact present up to the papillary dermis, in an area of up to around 1 mm from the external surface of the skin.

The part immediately below the epidermis is not generally considered a typical area for the giving of injections, perhaps also due to its limited extension in depth and thus the difficulty of injecting fluids precisely and reproducibly into this part. Moreover, the lack of vascularisation could lead to believe that fluids injected at this depth cannot be absorbed quickly or effectively; and finally, with syringes of the conventional type, a much reduced depth of injection may cause the leakage of the injected fluid through the incision produced by the needle when it is withdrawn.

The inventors have, however, noted that the injections just under the epidermis (at a depth from the external surface of the skin of between around 0.8 and 1.2 mm, depending on the person and the area of the body) are effective, because the capillaries immediately below, while having smaller sections and thus reduced blood flow, also have an extremely reduced wall thickness, which favours the passage of the fluids from the surrounding tissues to the vessel and thus into the bloodstream. These injections, thanks to the area wherein they occur, prevent in advance the problems discussed in the introduction, namely the possible severing of blood vessels (resulting in blood loss and inflammation) and contact with nerve endings. Moreover, injections in the area immediately below the epidermis are effective in the case of dermatological and cosmetic treatments, for example for the injection of fillers such as hyaluronic acid-based compositions.

Also the other problems that have to date limited the use of injections in the area immediately below the epidermis are resolved with the device or assembly of the present invention; in fact, by means of the latter, by an appropriate setting of the dimensions of the central part 13 and the positioning of the needle tip inside the device of the invention, it is possible to precisely control the depth reached by the needle tip with respect to the external surface of the skin. In addition, the lateral compression exerted by the lobes 15 causes the area between the lobes to undergo a slight upward deformation (i.e. towards the device according to the invention), making it to assume a slightly convex shape; when the needle is withdrawn, the lateral pressure of the lobes is released, and the skin in the area surrounding the injection site relaxes and returns to its natural arrangement; in this movement, the needle entry hole is closed with a movement opposite to that which caused the convexity, which presses the tissues and therefore the injected fluid towards the interior of the skin, thus preventing their emergence.

## Claims

1. Device (10; 50) to be applied to a needle holder or a syringe for the reduction of the pain caused by injections and the control of the injection depth, consists of a hollow body having essentially axial symmetry and that, in a sectional view along the axis of symmetry, shows in order: a first part (11; 61; 71; 81; 97) shaped internally so as to be able to be firmly attached to a needle holder, a syringe, or an adapter (90) attached to a needle holder or a syringe; a second central part (13), elastically deformable between two fixed positions of maximum and minimum extension, connected to said first part; and a third part (14), connected to said central part, consisting of at least three lobes (15) facing inwards, separated from each other by grooves (16) and having an opening (18) on the part opposite to said central part; said device being such that, at rest, said central part presents a first axial length **H** and said opening presents a first diameter value **D,** while when the device is compressed, said central part presents a second axial length **h < H** and said opening presents a second diameter value **d < D.**

2. Device according to claim 1, wherein the number of said lobes (15) is between six and twelve.

3. Device according to either of claims 1 or 2, wherein said lobes are inclined towards the axis of the device at an angle of between 30 and 60°.

4. Device according to any of the preceding claims, wherein said second part is formed of a deformable strip (30), shaped so as to present, in a sectional view, a concavity towards the inside of the device and a convexity towards the outside of the device.

5. Device according to any of claims 1 to 3, wherein said second part is formed of a cylindrical part with a series of cuts (31) parallel to each other and to the axis of the cylinder, with predefined fold lines (32) transverse to the cuts, so as to form parallel bands, each consisting of two parts (33, 33') separated by the fold lines.

6. Device according to any of claims 1 to 3, wherein said second part is formed of a bellows (34).

7. Device according to any of claims 1 to 6, wherein said first part (11) is fixed to said needle holder (40') or syringe by coupling, by means of an annular protuberance (41) formed on the external surface of said needle holder or syringe, that fits into a groove (42) of corresponding shape in the internal surface of said first part (11), or by means of an annular protuberance formed in the internal surface of said first part that fits into a groove of corresponding shape present on the external surface of said needle holder or syringe.

8. Device according to any of claims 1 to 6, wherein said first part (11) is fixed to said needle holder (40") or syringe by pressure.

9. Device according to any of claims 1 to 6, wherein said first part is fixed to said needle holder or syringe by means of a coupling of the Luer-lok type.

10. Device according to any of claims 1 to 6, wherein said first part (61) is mounted in an adjustable manner on said needle holder (63) or syringe, having a cylindrical external surface, by means of an annular protuberance (64) formed on the external surface of said needle holder or syringe, which fits into one of a series of grooves (62, 62', 62", ...) with a corresponding shape of a series of grooves, said grooves being arranged at different heights along the internal surface of said first part, or by means of an annular protuberance formed in the internal surface of said first part that fits into one of a series of grooves of corresponding shape of a series of grooves, present on the external surface of said needle holder or syringe.

11. Device according to any of claims 1 to 6, wherein said first part is mounted in an adjustable manner on said needle holder or syringe by means of an external thread on said needle holder (or syringe) having a cylindrical external surface, and a counterthread on the internal surface of the first part of the device.

12. Device according to any of claims 1 to 6, wherein said first part (71) is mounted in an adjustable manner on said needle holder or syringe, having a cylindrical external surface, by means of a protuberance (74) on the external surface of said needle holder (73) or syringe, that slides with friction into an oblique groove (72) in the surface of said first part, or by means of a protuberance on the internal surface of said first part that slides with friction into a groove, that is not a pass-through one, on the external surface of said needle holder or syringe.

13. Device according to any of claims 1 to 6, wherein said first part (81) is mounted in an adjustable manner on said needle holder or syringe, having a cylindrical external surface, by means of a protuberance (84) on the external surface of said needle holder (73) or syringe, that slides with friction into an oblique groove (82) in the surface of said first part, or by means of a protuberance on the internal surface of said first part that slides with friction into a groove that is not a pass-through one on the external surface of said needle holder or syringe, wherein said groove has a width that is not constant and has a series of equidistant narrowed sections (83, 83', 83", ...) such as to subdivide the groove into housings with a section equal to that of said protuberance.

14. Device according to any of the preceding claims, fixed on said needle holder or syringe by means of an adapter (90) comprising two semicylindrical parts (91, 91') joined together along a fold line (92), and having, on opposite sides of said fold line, elements (94, 94', 95, 95') for the closure of the adapter, said adapter being such that, when it is closed around said needle holder (96) or syringe, it cannot move axially along them, and having protuberances on its external surface to accommodate one of a series of grooves on the internal surface of said first part (97) of the device according to the invention.

15. Device according to any of the preceding claims, wherein the compression of said central part (13) causes the emergence of the needle tip (19) from said opening (18) at a value of between 0.8 mm and 4 cm.
